# EUROPÄISCHE PATENTSCHRIFT

(11) **EP 3 454 825 B1**
(45) Veröffentlichungstag und Bekanntmachung des Hinweises auf die Patenterteilung: **06.05.2026**
(21) Anmeldenummer: 16726805.1
(22) Anmeldetag: 14.05.2016
(51) Int. Cl.: A61K 31/222, A61K 8/34, A61Q 11/00, A61K 31/05, A61K 31/045, A61K 8/37

(54) **MENTHOL-HALTIGE AROMAZUBEREITUNGEN**
AROMA PREPARATIONS CONTAINING MENTHOL
PRÉPARATIONS D'AROMA CONTENANT DE MENTHOL

(43) Veröffentlichungstag der Anmeldung: 20.03.2019
(60) Teilanmeldung: 26170117.1
(73) Patentinhaber: Symrise AG, 37603 Holzminden (DE)
(72) Erfinder: SIEGEL, Sven, 37671 Höxter (DE); MACHINEK, Arnold, 37603 Holzminden (DE); JOIN, Benoit, 37603 Holzminden (DE)
(74) Vertreter: Daniels, Stefanie Lisa
(86) Internationale Anmeldenummer: PCT/EP2016/060932
(87) Internationale Veröffentlichungsnummer: WO 2017/198284

(56) Entgegenhaltungen:
- CN-A- 103 451 017
- CN-A- 104 606 100
- JP-A- H07 145 598
- US-A- 5 711 937
- US-A1- 2009 226 549
- US-A1- 2015 313 955
- SCHMIDT E ET AL: "Chemical composition, olfactory evaluation and antioxidant effects of essential oil from Mentha x piperita", NATURAL PRODUCT COMMUNICATIONS, NATURAL PRODUCT INC, US, vol. 4, no. 8, 1 January 2009 (2009-01-01), pages 1107 - 1112, XP009188168, ISSN: 1934-578X
- DATABASE WPI 6 June 1995 Derwent World Patents Index; AN 1995-237508, ARAI H; MIYAGAWA K; OTAKI T; SEKIGUCHI K; TAKAHASHI J: "Aromatic sheet for use in cars, clothing, rooms, etc. - comprises fibrous base contg. e.g. eucalyptus, peppermint or rosemary oil covered with barrier material"
- DATABASE GNPD [online] MINTEL; 1 November 2014 (2014-11-01), MISTINE: "Mistine Dentiss", XP002760753, retrieved from GNPD Database accession no. 2760425

## Beschreibung

### GEBIET DER ERFINDUNG

Die Erfindung befindet sich auf dem Gebiet der Aromastoffe und betrifft neue Zubereitungen mit ausgewählten Aroma- und Kühlstoffen sowie orale Zubereitungen, die diese Mischungen enthalten.

### TECHNOLOGISCHER HINTERGRUND

4-Allyl-2-methoxyphenol, das auch als Eugenol bezeichnet wird, stellt einen bekannten Duft- und Aromastoff mit warm-würziger Note dar, der Bestandteil verschiedener ätherischer Öle, wie Nelkenöl (70 bis 95 Gew.-%), Piment- und Pimentblätteröl (60 bis 90 Gew.-%), Bayöl (50 bis 60 Gew.-%) oder der Gruppe der Zimtöle (5 bis 95 Gew.-%) ist. Außerdem findet er sich in Lorbeer, Basilikum, Bananen, Kirschen und Muskat. Eugenol und speziell (Gewürz- )Nelkenöl werden häufig in oralen Zubereitungen, speziell Mundpflegeprodukten eingesetzt, da es über antivirale, antibakterielle und anti-oxidative Eigenschaften verfügt. So verbessern beispielsweise geringe Mengen Eugenol die antibakteriellen Eigenschaften von Thymol und Terpineol **(**EP 2480090 B1**,** UNILEVER)

Nachteilig ist jedoch, dass viele Menschen den Geschmack des Eugenols bzw. Nelkenöls ablehnen, da sie ihn mit dem unangenehmen Besuch beim Zahnarzt assoziieren. Eugenol ist nämlich auch fester Bestandteil des so genannten Zinkoxid-Eugenol-Zements, der häufig in der Zahnheilkunde eingesetzt wird. Das gleiche gilt für strukturell verwandte Stoffe, wie beispielsweise Anethol, Thymol oder Carvacol.

Ein weiterer Nachteil dieser Stoffe besteht ferner darin, dass sie nicht nur einen gewünschten warm-würzigen Geschmackseindruck erzeugen können, sondern von vielen Verbrauchern auch als brennend und sogar schmerzhaft empfunden wird.

### STAND DER TECHNIK

US 2015313955 A1 (POTNIS) betrifft eine therapeutische Zusammensetzung zur Behandlung von Zahnschmerzen, die das Öl der Gewürznelke bzw. Eugenol und Campher sowie einen physiologischen Kühlstoff enthält.

### AUFGABE DER ERFINDUNG

Die Aufgabe der vorliegenden hat daher darin bestanden, den Geschmackseindruck der genannten Stoffe bzw. ätherischen Ölen, die die diese enthalten, so zu modifizieren, dass der warm-würzige Eindruck verstärkt und der brennend-schmerzhafte Eindruck unterdrückt wird.

### BESCHREIBUNG DER ERFINDUNG

Ein erster Gegenstand der Erfindung betrifft daher Aromazubereitungen enthaltend
(a) eine Verbindung der Formel (I) 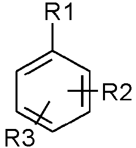 in der R1 für einen linearen oder verzweigten, gesättigten oder ungesättigten Kohlenwasserstoffrest mit 1 bis 4 Kohlenstoffatomen, R2 für Wasserstoff oder eine Hydroxylgruppe und R3 für Wasserstoff, eine Methyl- oder Methoxygruppe stehen, oder ein ätherisches Öl mit einem Gehalt an einer oder mehrerer Verbindungen der Formel (I),
(b1) Menthol und
(b2) Menthylacetat,
   worin
   - die Komponenten (a) und (b1+b2) im Gewichtsverhältnis von 80:20 bis 99:1 und
   - die Komponenten (b1) und (b2) im Gewichtsverhältnis 80:20 bis 20:80
   vorliegen.

Überraschenderweise wurde gefunden, dass der Zusatz von Mentholverbindungen, insbesondere von Menthol, Menthylacetat und ganz besonders bevorzugt von Mischungen aus Menthol und Menthylacetat, die eingangs formulierte Aufgabe in vollem Umfang erfüllen. Die Mischungen weisen nach wie vor einen warm-würzigen Eindruck auf, der jedoch nicht mehr brennend oder sogar schmerzhaft ist und nur noch entfernt mit einem Zahnarztbesuch assoziiert werden kann.

### Verbindungen der Formel (I) und ätherische Öle

Im Sinne der vorliegenden Erfindung sind solche Verbindungen der Formel (I) bevorzugt, bei denen R1 für eine -C(CH₃)₂, eine -CH₂-CH=_{CH2} oder -CH=CH-CH₃ Gruppe steht. Ebenfalls strukturell bevorzugt ist, dass R2 und R3 nicht beide gleichzeitig für Wasserstoff stehen.

Konkrete Ausführungsformen für die Verbindungen der Formel (I) sind:

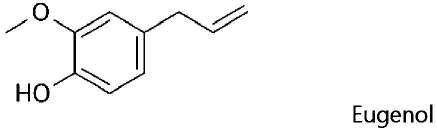

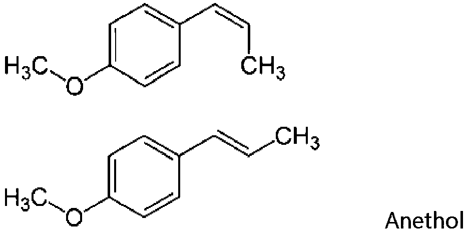

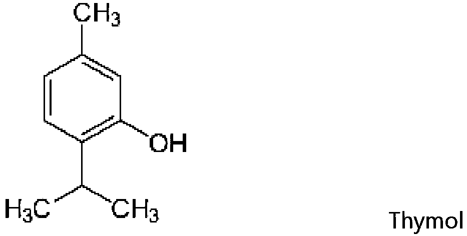

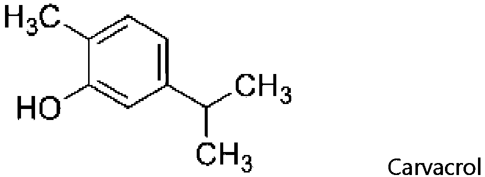

Neben dem Grundkörper Eugenol kommen als Komponente (a) des Weiteren auch die eingangs schon genannten ätherischen Öle, also Pimentöl, Pimentblätteröl, Bayöl, Zimtöl, Lorbeeröl, Muskatöl und insbesondere Gewürznelken oder kurz Nelkenöl in Betracht.

Anethol ist Bestandteil beispielsweise des Anisöls. Thymol und sein Isomer Carvacrol finden sich als Bestandteile des ätherischen Öls von Thymian, Oregano und Bohnenkraut.

Zur Verdeutlichung sei ausgeführt, dass anstelle der einzelnen Verbindungen der Formel (I) auch Gemische, insbesondere Gemische von Eugenol, Anethol, Thymol und/oder Carvacrol eingesetzt werden können. Gleiches gilt für ätherische Öle - wie beispielsweise die oben genannten - die eine oder mehrere Verbindungen der Formel (I), speziell Eugenol, Anethol, Thymol und/oder Carvacrol in praktisch beliebigen Mengenverhältnissen.

### Menthol und Mentholverbindungen

Mentholverbindungen die im Sinne der Erfindung als Komponente (b) eingesetzt werden können, sind Menthol und Menthylacetat.

Weitere Mentholverbindungen, die im Zusammenhang mit der Erfindung als Komponente (b) offenbart, jedoch nicht beansprucht werden sind ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutarate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und - amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter der dieser Stoffe stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 (V.Mane Fils) ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

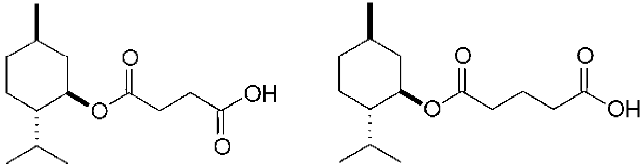

Beispiele für Anwendungen dieser Stoffe finden sich beispielsweise in den Druckschriften WO 2003 043431 (Unilever) oder EP 1332772 A1 (IFF).

Die nächste wichtige Gruppe umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten:

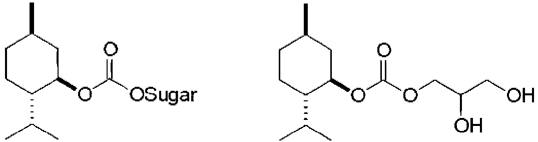

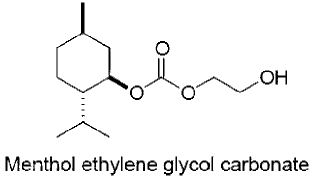

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Ebenfalls geeignet, weenn auch nicht beansprucht, sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird.

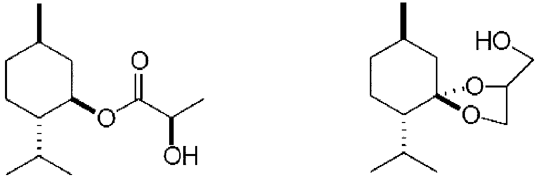

Erstere Struktur wird durch Veresterung von Milchsäure mit Menthol, letztere durch Acetalisierung von Menthon mit Glycerin gewonnen (vgl. DE 2608226 A1**,** H&R). In diese Gruppe von Verbindungen gehört auch das 3-(l-Menthoxy)-1,2,propandiol, das auch als Cooling Agent 10 bekannt ist (FEMA GRAS 3784, vgl. US 6,328,982**,** TIC), sowie das 3-(I-Menthoxy)-2-methyl-1,2,propandiol (FEMA GRAS 3849), das über eine zusätzliche Methylgruppe verfügt.

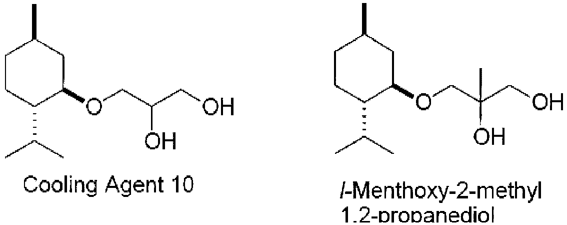

Die Herstellung des 3-(l-Menthoxy)-1,2,propandiol erfolgt beispielsweise ausgehend von Menthol nach dem folgenden Schema (vgl. US 4,459,425, Takagaso):

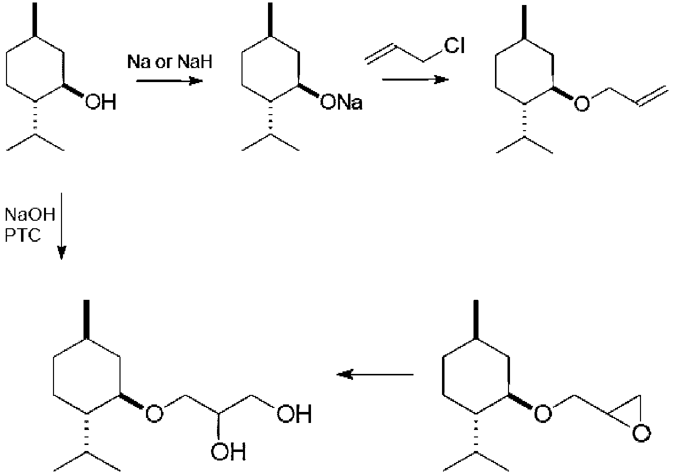

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

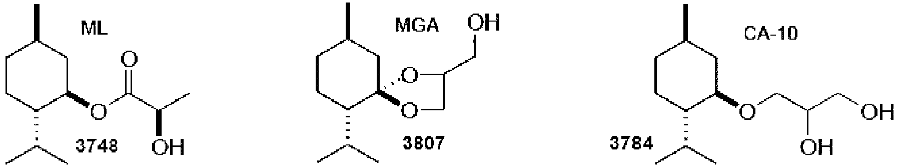

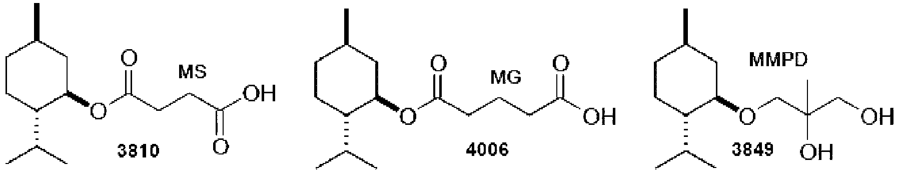

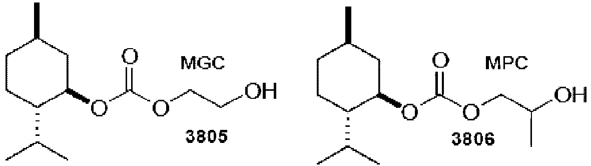

Als ebenfalls geeignet, wenn auch nicht beansprucht, haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

Ebenfalls geeignet sind die beiden Kühlstoffe EC-180 (FEMA GRAS 4496), EC-190 (FEMA GRAS 4549):

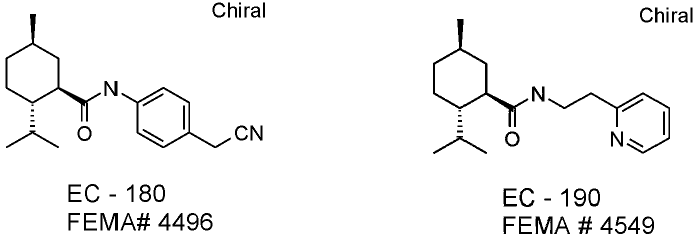

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

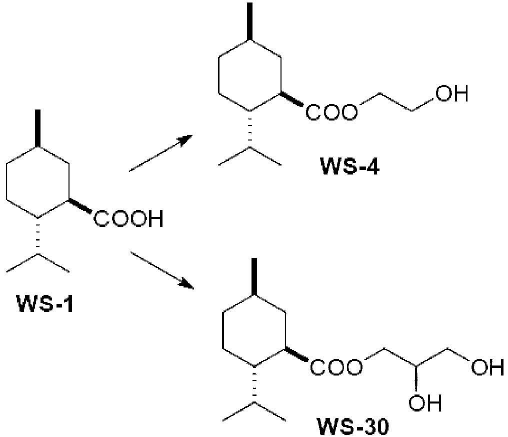

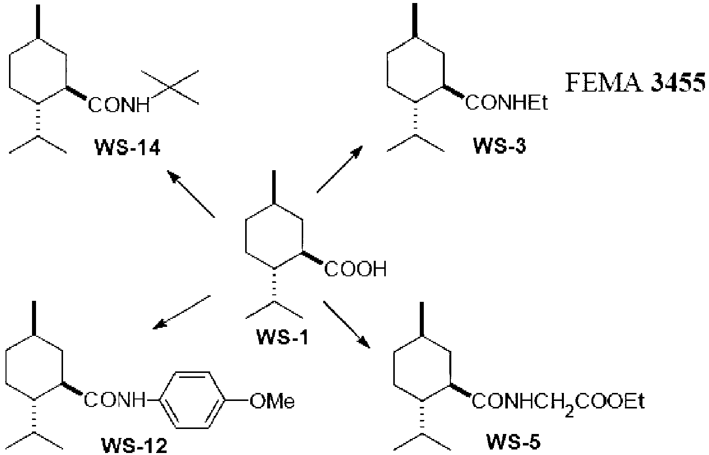

Die sich von WS-1 ableitenden Ester werden beispielsweise in US 4,157,384**,** die entsprechenden N-substituierten Amide in J. Soc. Cosmet. Chem. S. 185-200 (1978**)** beschrieben.

### Aromazubereitungen

In einer bevorzugten Ausführungsform können die Zubereitungen die Komponenten (a) und (b1+b2) im Gewichtsverhältnis von etwa 60:40 bis etwa 40:60 enthalten. Bezüglich der Mischung aus Menthol und Menthylacetat eingesetzt, kann das Gewichtsverhältnis der beiden Stoffe (b1) und (b2) vorteilhafterweise im Bereich von etwa 60:40 bis etwa 40:60 liegen.

Eine ebenfalls vorteilhafte Aromazubereitungen, die zwar hiermit offenbart, aber nicht beansprucht wird umfasst:
(a) 80 bis 99 Gew.-% Verbindungen der Formel (I) oder ein ätherisches Öl mit einem Gehalt an einer oder mehrerer Verbindungen der Formel (I),
(b1) 1 bis 20 Gew.-% Menthol und
(b2) 1 bis 20 Gew.-% Menthylacetat
mit der Maßgabe, dass sich die Mengenangaben jeweils zu 100 Gew.-% ergänzen. Mit dieser Einschränkung wird klargestellt, dass alle Mischungsverhältnisse, die sich nicht zu 100 Gew.-% addieren, ausgeschlossen sind und der Fachmann innerhalb der Vorgaben beliebige Mischungsverhältnisse auswählen kann, die die oben geschilderte Lehre erfüllen, ohne hierzu erfinderisch tätig werden zu müssen. Im Übrigen wird auf die Ausführungsbeispiele verwiesen.

### GEWERBLICHE ANWENDBARKEIT

Ein weiterer Gegenstand der vorliegenden Erfindung betrifft Zubereitungen zur oralen Aufnahme, enthaltend die oben beschriebenen Aromamischungen enthalten. Bei diesen Zubereitungen kann es sich sowohl um Nahrungsmittel, pharmazeutische Zubereitungen oder Mund- und Zahnpflegemittel handeln, wobei der Übergang zwischen diesen Gruppen fließend ist. Ein Kaubonbon kann beispielsweise ebenso als Süßigkeit als auch als Erkältungsmittel Verwendung finden, ein Kaugummi ebenfalls als Erfrischung oder eben zur medizinischen Zahnpflege dienen.

### Orale Zubereitungen

Typische Beispiele für Nahrungsmittel, die die erfindungsgemäßen Zubereitungen enthalten, sind Hartkaramellen, Kaubonbons oder Kaugummis. Typische Beispiele für pharmazeutische Zubereitungen, die die erfindungsgemäßen Zubereitungen enthalten, sind Erkältungssäfte, Erkältungssprays oder Erkältungsbonbons. Typische Beispiele für pharmazeutische Zubereitungen, die die erfindungsgemäßen Zubereitungen enthalten, sind Zahnpasten, Mundwässer oder medizinische Kaugummis.

Die genannten oralen Zubereitungen können die Aromamischungen in Mengen von etwa 0,5 bis etwa 5 Gew.-% und insbesondere etwa 1 bis etwa 2,5 Gew.-% - bezogen auf die Endzubereitung - enthalten. Im Folgenden werden Hilfs- und Zusatzstoffe beschrieben, die die oralen Zubereitungen ebenfalls enthalten können.

### Nahrungsmittel

Nahrungsmittel, welche die erfindungsgemäßen Aromamischungen enthalten, stellen in der Regel Hartkaramellen oder Kau- bzw. Lutschbonbons dar. Der wichtigste Zusatzstoff für diese Produkte sind Süßungsmittel, darunter vor allem auch solche, die nicht zuckerbasiert sind. Daneben sind vor allem noch weitere Aromen und Lebensmittelfarbstoffe zu nennen.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte.

### Weitere Aromastoffe

Die Erfindung erlaubt in den oralen Zubereitungen insbesondere auch den Einsatz von weiteren Aromastoffen mit Ester-, Aldehyd- oder Lactonstruktur, die in Gegenwart von Titandioxid und unter Lichteinfluss besonders schnell abgebaut werden. Die Erfindung sorgt somit auch für eine verbesserte Stabilität, speziell Lagerstabilität der Aromastoffe.

Die erfindungsgemäßen oralen Zubereitungen können einen oder mehrere zusätzliche Aromastoffe enthalten. Typische Beispiele umfassen: Acetophenon, Allylcapronat, alphalonon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### Geschmacksverstärker

Die oralen Zubereitungen können auch Stoffe zum Verstärken eines salzigen, gegebenenfalls leicht sauren und/oder Umami-Geschmackseindrucks enthalten. Es werden somit die erfindungsgemäßen Produkte bzw. Aromamischungen in Kombination mit zumindest einer weiteren zur Verstärkung eines an-genehmen Geschmackseindrucks (salzig, Umami, gegebenenfalls leicht sauer) geeigneten Substanz verwendet. Hierbei bevorzugt sind salzig schmeckende Verbindungen und salzver-stärkende Verbindungen. Bevorzugte Verbindungen sind in der WO 2007/045566 offenbart. Ferner bevorzugt sind Umami-Verbindungen wie in der WO 2008/046895 und EP 1 989 944 beschrieben sind.

Weiterhin können erfindungsgemäß bevorzugte Produkte auch Aromastoffe zur Maskierung von bitteren und/oder adstringierenden Geschmackseindrücken umfassen (Geschmackskorrigentien). Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-tri-,hydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Bevorzugte Aromastoffe sind solche, die einen süßen Geruchseindruck verursachen, wobei der oder die weiteren Aromastoffe, die einen süßen Geruchseindruck verursachen, bevor-zugt ausgewählt sind aus der Gruppe bestehend aus:

Vanillin, Ethylvanillin, Ethylvanillinisobutyrat (= 3 Ethoxy-4-isobutyryloxybenzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

### Wirkstoffe zur Maskierung von unangenehmen Geschmackseindrücken

Weiterhin können die oralen Zubereitungen auch Stoffe umfassen, die ebenfalls zur Maskierung von bitteren und/oder adstringierenden Geschmackseindrücken dienen. Diese weiteren Geschmackskorrigenzien werden z. B. aus der folgenden Liste ausgewählt: Nucleotiden (z.B. Adenosin-5'-monophosphat, Cytidin-5'-monophosphat) oder deren physiologisch akzeptablen Salzen, Lactisolen, Natriumsalzen (z.B. Natriumchlorid, Natriumlactat, Natriumcitrat, Natriumacetat, Natriumgluconoat), Hydroxyflavanonen, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1258200 A2). Hydroxybenzoesäureamiden, dabei vorzugsweise 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid*,* 2-Hydroxy-benzoesäure*-N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxy-benzyl)amid-Mono-natriumsalz, 2,4-Dihydroxybenzoesäure-*N*-2-(4-hydroxy-3-methoxy-phenyl)ethylamid, 2,4-Dihydroxybenzoesäure-*N*-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamiden (insbesondere solche wie beschrieben in WO 2006/024587); Hydroxydeoxybenzoinen, dabei vorzugsweise 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)-ethanon und 1-(2-Hydroxy-4-methoxyphenyl)-2-(4-hydroxy-3-methoxyphenyl)ethanon) (insbesondere solche wie beschrieben in WO 2006/106023 beschrieben, die bezüglich der darin offenbarten entsprechenden Verbindungen auf dem Wege der Verweisung Bestandteil dieser Anmeldung wird); Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527); Diacetyltrimeren; gamma-Aminobuttersäuren (insbesondere solche wie beschrieben in WO 2005/096841); Divanillinen (insbesondere solche wie beschrieben in WO 2004/078302) und 4-Hydroxydihydrochalconen (vorzugsweise wie beschrieben in US 2008/0227867 A1), dabei insbesondere Phloretin und Davidigenin, Aminosäuren oder Gemische von Molkeproteinen mit Lecithinen, Hesperetin wie in der WO 2007/014879 offenbart, 4-Hydroxydihydrochalkonen wie in der WO 2007/107596 offenbart, oder Propenylphenylglycosiden (Chavicolglycosiden) wie in EP 1955601 A1 beschrieben, oder Extrakten aus *Rubus suavissimus,* Extrakte aus *Hydrangea macrophylla* wie in EP 2298084 A1 beschrieben, Pellitorin und abgeleiteten Aromakompositionen wie in EP 2008530 A1 beschrieben, Umami-Verbindungen wie in WO 2008/046895 A1 und EP 1989944 A1 beschrieben, Umami-Verbindungen wie beschrieben in EP 2064959 A1 bzw. EP 2135516 A1, Vanillyllignanen, Enterodiol, sowie N-Decadienoylaminosäuren und deren Gemische.

### Lebensmittelfarbstoffe

Lebensmittelfarbstoffe oder kurz Farbstoffe sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### Kaugummis

Bei den bevorzugten oralen Zubereitungen kann es sich insbesondere auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**.**

Zusätzlich zu der wasserunlöslichen Gummibasis enthalten Kaugummizubereitungen regelmäßig einen wasserlöslichen Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Mund- und Zahnpflegemitttel

Erfindungsgemäße oral konsumierbare süß schmeckende Produkte können insbesonere der Mund- und Zahnreinigung und-pflege dienen. Beispiele hierfür sind wie gesagt Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern- , Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Verfahren und Verwendung

Ein weiterer Gegenstand der Erfindung betrifft ein Verfahren zur Verstärkung des warm-würzigen und Verminderung des brennend, schmerzhaften Geschmacks von Verbindungen der Formel (I) (Komponente a) enthaltenden ätherischen Ölen dadurch gekennzeichnet, dass man Zubereitungen, die diese Stoffe enthalten, eine Mischung aus Menthol (Komponente b1) und Menthylacetat (Komponente b2) zusetzt, wobei im besagten ätherischen Öl
- die Komponenten (a) und (b1+b2) im Gewichtsverhältnis von 80:20 bis 99:1 und
- die Komponenten (b1) und (b2) im Gewichtsverhältnis 80:20 bis 20:80
vorliegen.

Ebenfalls beansprucht wird die Verwendung einer Mischung aus Menthol (Komponente b1) und Menthylacetat (Komponente b2) zur Verstärkung des warm-würzigen und Verminderung des brennend, schmerzhaften Geschmacks von Verbindungen der Formel (I) (Komponente a) enthaltenden ätherischen Ölen, wobei im besagten ätherischen Öl
- die Komponenten (a) und (b1+b2) im Gewichtsverhältnis von 80:20 bis 99:1 und
- die Komponenten (b1) und (b2) im Gewichtsverhältnis 80:20 bis 20:80
eingesetzt werden.

Soweit Verfahren und Verwendung wie oben erläutert betroffen sind, gelten die gleichen Mengenangaben und bevorzugten Ausführungsformen wie vorstehend ausführlich beschrieben mit, ohne dass es hierzu einer Wiederholung bedarf.

### BEISPIELE

### ANWENDUNGSTECHNISCHE UNTERSUCHUNGEN

### Beispiele 1 und 2, Vergleichsbeispiele V1 bis V4

Das geschmackliche Profil verschiedener Aromamischungen wurde von einem Panel bestehend aus 5 geschulten Testern beurteilt. Dazu wurden die Mischungen in einer Menge von 0,5 Gew.-% in eine ungezuckerte und nicht aromatisierte Standard-Kaubonbonzubereitung eingearbeitet. Die Ergebnisse sind in **Tabelle 1** wiedergegeben. Die Bewertung erfolgte gemäß der Benotung (5) = trifft sehr zu bis (0) = trifft weitgehend nicht zu. Angegeben sind die Mittelwerte. Die Beispiele 1 und 2 sind erfindungsgemäß, die Beispiele V1 bis V4 dienen zum Vergleich.

**Tabelle 1**

| Sensorische Beurteilung von Aromamischungen | | | | | | |
|---|---|---|---|---|---|---|
| **Komponenten** | **V1** | **V2** | **V3** | **V4** | **1** | **2** |
| Eugenol | 100 | - | 80 | 80 | 80 | - |
| Nelkenöl | - | 100 | - | - | - | 80 |
| Menthol | - | - | 20 | - | 10 | 10 |
| Menthylacetat | - | - | - | 20 | 10 | 10 |

| ***Sensorische Beurteilung*** | | | | | | |
|---|---|---|---|---|---|---|
| Warm-würziger Geschmack | 4,2 | 4,1 | 4,5 | 4,3 | 4,5 | 4,8 |
| Brennender Geschmack | 4,5 | 4,5 | 3,1 | 2,8 | 2,1 | 2,0 |
| Schmerzhafte Empfindung | 3,0 | 2,9 | 1,5 | 1,5 | 1,0 | 1,0 |

Die Beispiele und Vergleichsbeispiele zeigen, dass die erfindungsgemäßen Mischungen sensorisch deutlich besser als die Einzelsubstanzen beurteilt werden. Im Folgenden werden eine Reihe von Formulierungsbeispielen gegeben.

### FORMULIERUNGSBEISPIELE

### Beispiel 1

Zahnpasta (Mengenangaben als Gew.-%)

| **KOMPONENTEN** | **MENGE** |
|---|---|
| Water (deionized) | Ad 100 |
| Sorbitol 70% | 45.00 |
| Trisodiumphosphate | 0.10 |
| Saccharin | 0.20 |
| Sodiummonofluorophosphate | 1.14 |
| PEG 1500 | 5.00 |
| Sident 9 (abrasive silica) | 10.00 |
| Sident 22 S (Thickening silica) | 8.00 |
| Sodiumcarboxymethylcellulose | 1.10 |
| Titanium (IV) oxide | 0.50 |
| Water (deionized) | 4.50 |
| Sodiumlaurylsulfate (SLS) | 1.50 |
| Aromamischung | 1.00 |
| Solbrol M (Sodium salt) (Methylparaben) | 0.15 |
| Aromamischung 3 | 0.40 |

### Beispiel 2

Zahnpasta mit Zinkcitrat (Mengenangaben als Gew.-%)

| **KOMPONENTEN** | **MENGE** |
|---|---|
| Water (deionized) | Ad 100 |
| Sorbitol 70% | 45.00 |
| Trisodiumphosphate | 0.10 |
| Saccharin | 0.20 |
| Sodiummonofluorophosphate | 1.14 |
| PEG 1500 | 5.00 |
| Sident 9 (abrasive silica) | 10.00 |
| Sident 22 S (Thickening silica) | 8.00 |
| Sodiumcarboxymethylcellulose | 1.10 |
| Zinc citrate | 1.00 |
| Titanium (IV) oxide | 0.50 |
| Water (deionized) | 4.50 |
| Sodiumlaurylsulfate (SLS) | 1.50 |
| Aromamischung | 1.00 |
| SymDiol^{®} 68 (1.2-Hexanediol. Caprylylglycol) | 0.25 |
| Aromamischung 4 | 0.10 |

### Beispiel 3

Mundwasser (Mengenangabe als Gew.-%)

| **KOMPONENTEN** | **MENGE** |
|---|---|
| Ethylalcohol | 10.00 |
| Cremophor CO 40 (PEG 40 hydrogenated castor oil) | 1.00 |
| Aromamischung | 0.25 |
| Water (deionized) | To 100.00 |
| Sorbitol 70% | 5.00 |
| Sodiumsaccharin 450 | 0.07 |
| Sodiumfluoride | 0.18 |
| Benzoic acid | 0.12 |
| Aromamischung 3 | 0.30 |

### Beispiel 4

Dentalgel (Mengenangaben als Gew.-%)

| **KOMPONENTEN** | **MENGE** |
|---|---|
| Na carboxymethylcellulose | 0.40 |
| Sorbitol 70 %. in water | 72.00 |
| Polyethylene glycol (PEG) 1500 | 3.00 |
| Na saccarinate | 0.07 |
| Na fluoride | 0.24 |
| Aromamischung | 1.00 |
| Abrasive silica | 11.00 |
| Thickening silica | 6.00 |
| Sodium dodecyl sulfate (SDS) | 1.40 |
| Dist. water | Ad 100 |
| p-Hydroxybenzoic acid (PHB) ethyl ester | 0.15 |
| Aromamischung 4 | 0.20 |

### Beispiel 5

Anti-Plaque Zahncreme (Mengenangaben als Gew.-%)

| **KOMPONENTEN** | **MENGE** |
|---|---|
| Carrageenan | 0.90 |
| Glycerol | 15.00 |
| Sorbitol 70 %. in water | 25.00 |
| PEG 1000 | 3.00 |
| Na fluoride | 0.24 |
| Tetrapotassium diphosphate | 4.50 |
| Tetrasodium diphosphate | 1.50 |
| Na saccarinate | 0.40 |
| Precipitated silica | 20.00 |
| Titanium dioxide | 1.00 |
| Triclosan | 0.30 |
| Spearmint flavor (comprising 60 wt.% I-carvone and 25 wt.% I-menthol) | 1.00 |
| Sodium dodecyl sulfate | 1.30 |
| Dist. water | Ad 100 |
| Benzylalcohol | 0.50 |
| Aromamischung 3 | 0.25 |

### Beispiel 6

Zahncreme für empfindliche Zähne (Mengenangaben als Gew.-%)

| **KOMPONENTEN** | **MENGE** |
|---|---|
| Na carboxymethylcellulose | 0.70 |
| Xanthan gum | 0.50 |
| Glycerol | 15.00 |
| Sorbitol 70 %. in water | 12.00 |
| Potassium nitrate | 5.00 |
| Sodium monofluorophosphate | 0.80 |
| Na saccharinate | 0.20 |
| Aromamischung | 1.00 |
| Ca-carbonate | 35.00 |
| Silicon dioxide | 1.00 |
| Sodium dodecyl sulfate (SDS) | 1.50 |
| Dist. water | Ad 100 |
| PHB methyl ester and PHB propyl ester | 0.20 |
| Aromamischung 4 | 0.50 |

### Beispiel 7

Zahncreme und Mundwasser 2:1 (Mengenangaben als Gew.-%)

| **KOMPONENTEN** | **MENGE** |
|---|---|
| Sorbitol | 40.00 |
| Glycerol | 20.00 |
| Ethanol | 5.00 |
| Water | Ad 100 |
| Na monofluorophosphate | 0.75 |
| Saccharin | 0.20 |
| Sident 9 (abrasive silicon dioxide) | 20.00 |
| Sident 22 S (thickening silicon dioxide) | 2.00 |
| Sodium carboxymethylcellulose | 0.30 |
| Sodium lauryl sulfate (SDS) | 1.20 |
| Color (Suspension. 1% in water) C.I. Pigment Blue 15 | 0.50 |
| Aromamischung | 0.90 |
| Solbrol M. sodium salt (methylparaben. sodium salt) | 0.20 |
| Aromamischung 3 | 0.30 |

### Beispiel 8

Mundwasser mit Fluorid (Mengenangaben als Gew.-%)

| **KOMPONENTEN** | **MENGE** |
|---|---|
| Ethanol | 7.00 |
| Glycerol | 12.00 |
| Na fluoride | 0.05 |
| Pluronic F-127^{®} (BASF. surface-active substance) | 1.40 |
| Na phosphate buffer pH 7.0 | 1.10 |
| Na saccharinate | 0.10 |
| Aromamischung | 0.15 |
| Chlorhexidine digluconate | 0.2 |
| Dist. water | to 100 |
| Sorbic acid | 0.20 |
| Aromamischung 4 | 0.30 |

### Beispiel 9

Zuckerfreies Kaugummi (Mengenangaben als Gew.-%)

| **KOMPONENTEN** | **MENGE** |
|---|---|
| Chewing gum base | 30.00 |
| Sorbitol. powder | Ad 100 |
| Palatinite | 9.50 |
| Xylitol | 2.00 |
| Mannitol | 3.00 |
| Aspartame | 0.10 |
| Acesulfame K | 0.10 |
| Emulgum / emulsifier | 0.30 |
| Sorbitol 70 %. in water | 14.00 |
| Glycerol | 1.00 |
| Aromamischung | 1.50 |
| Aromamischung 3 | 0.20 |

## Patentansprüche

1. Aromazubereitungen enthaltend
(a) eine Verbindung der Formel (I) 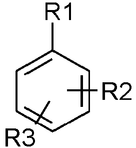 in der R1 für einen linearen oder verzweigten, gesättigten oder ungesättigten Kohlenwasserstoffrest mit 1 bis 4 Kohlenstoffatomen, R2 für Wasserstoff oder eine Hydroxylgruppe und R3 für Wasserstoff, eine Methyl- oder Methoxygruppe stehen, oder ein ätherisches Öl mit einem Gehalt an einer oder mehrerer Verbindungen der Formel (I),
(b1) Menthol und
(b2) Menthylacetat,
worin
- die Komponenten (a) und (b1+b2) im Gewichtsverhältnis von 80:20 bis 99:1 und
- die Komponenten (b1) und (b2) im Gewichtsverhältnis 80:20 bis 20:80
vorliegen.

2. Zubereitungen nach Anspruch 1, **dadurch gekennzeichnet, dass** die Verbindungen der Formel (I) ausgewählt sind aus der Gruppe, die gebildet wird von Eugenol, Anethol, Thymol und Carvacol sowie deren Gemischen.

3. Zubereitungen zur oralen Aufnahme, enthaltend die Mischungen nach Anspruch 1.

4. Zubereitungen nach Anspruch 3, **dadurch gekennzeichnet, dass** es sich um Nahrungsmittel, pharmazeutische Zubereitungen und/oder Mund- und Zahnpflegemittel handelt.

5. Zubereitungen nach Anspruch 3, **dadurch gekennzeichnet, dass** es sich um Hartkaramellen, Kaubonbons oder Kaugummis handelt.

6. Zubereitungen nach Anspruch 3, **dadurch gekennzeichnet, dass** es sich um Erkältungssäfte, Erkältungssprays oder Erkältungsbonbons handelt.

7. Zubereitungen nach Anspruch 3, **dadurch gekennzeichnet, dass** es sich um Zahnpasten, Mundwässer oder medizinische Kaugummis handelt.

8. Zubereitungen nach mindestens einem der Ansprüche 3 bis 7, **dadurch gekennzeichnet, dass** sie die Zubereitungen nach Anspruch 1 in Mengen von 0,5 bis 5 Gew.-% - bezogen auf die Endzubereitung - enthalten.

9. Verfahren zur Verstärkung des warm-würzigen und Verminderung des brennend, schmerzhaften Geschmacks von Verbindungen der Formel (I) (Komponente a) enthaltenden ätherischen Ölen **dadurch gekennzeichnet, dass** man Zubereitungen, die diese Stoffe enthalten, eine Mischung aus Menthol (Komponente b1) und Menthylacetat (Komponente b2) zusetzt, wobei im besagten ätherischen Öl
- die Komponenten (a) und (b1+b2) im Gewichtsverhältnis von 80:20 bis 99:1 und
- die Komponenten (b1) und (b2) im Gewichtsverhältnis 80:20 bis 20:80
vorliegen.

10. Verwendung einer Mischung aus Menthol (Komponente b1) und Menthylacetat (Komponente b2) zur Verstärkung des warm-würzigen und Verminderung des brennend, schmerzhaften Geschmacks von Verbindungen der Formel (I) (Komponente a) enthaltenden ätherischen Ölen, wobei im besagten ätherischen Öl
- die Komponenten (a) und (b1+b2) im Gewichtsverhältnis von 80:20 bis 99:1 und
- die Komponenten (b1) und (b2) im Gewichtsverhältnis 80:20 bis 20:80
eingesetzt werden.

## Claims

1. Aroma preparations comprising
(a) a compound according to formula (I) 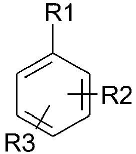 in which R1 represents a linear or branched, saturated or unsaturated hydrocarbon radical having 1 to 4 carbon atoms, R2 represents hydrogen or a hydroxyl group and R3 represents hydrogen, a methyl or methoxy group, or an essential oil containing one or more compounds of formula (I),
(b1) menthol and
(b2) menthyl acetate,
wherein
- components (a) and (b1 +b2) in a weight ratio of 80:20 to 99:1 and
- components (b1) and (b2) in a weight ratio of 80:20 to 20:80
are present.

2. Preparation according to Claim 1, **characterized in that** the compounds of formula (I) are selected from the group consisting of eugenol, anethole, thymol and carvacol and mixtures thereof.

3. Preparations for oral administration containing the mixtures according to claim 1.

4. Preparations according to Claim 3, **characterized in that** they are foodstuffs, pharmaceutical preparations and/or oral and dental care preparations.

5. Preparations according to Claim 3, **characterized in that** they are hard candies, chewy candies or chewing gums.

6. Preparations according to Claim 3, **characterized in that** they are cold juices, cold sprays or cold lozenges.

7. Preparations according to Claim 3, **characterized in that** they are toothpastes, mouthwashes or medicinal chewing gums.

8. Preparations according to at least one of Claims 3 to 7, **characterized in that** they contain the preparations according to claim 1 in amounts of 0.5 to 5% by weight - relative to the final preparation.

9. A process for enhancing the warm-spicy taste and reducing the burning, painful taste of essential oils containing compounds of formula (I) (component a), **characterized in that** a mixture of menthol (component b1) and menthyl acetate (component b2) is added to preparations containing these substances, wherein in the said essential oil
- components (a) and (b1 +b2) in a weight ratio of 80:20 to 99:1 and
- components (b1) and (b2) in a weight ratio of 80:20 to 20:80
are present.

10. The use of a mixture of menthol (component b1) and menthyl acetate (component b2) for enhancing the warm-spicy taste and reducing the burning, painful taste of essential oils containing compounds of the formula (I) (component a), wherein in said essential oil
- components (a) and (b1 +b2) in a weight ratio of 80:20 to 99:1 and
- components (b1) and (b2) in a weight ratio of 80:20 to 20:80
are used.

## Revendications

1. Préparations aromatiques comprenant
(a) un composé selon la formule (I) 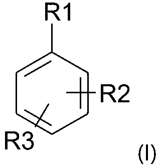 dans laquelle R1 représente un radical hydrocarboné saturé ou insaturé, linéaire ou ramifié, ayant de 1 à 4 atomes de carbone, R2 représente l'hydrogène ou un groupe hydroxyle et R3 représente l'hydrogène, un groupe méthyle ou méthoxy, ou une huile essentielle contenant un ou plusieurs composés de la formule (I),
(b1) menthol et
(b2) acétate de menthyle,
dans lequel
- les composants (a) et (b1+b2) dans un rapport en poids de 80:20 à 99:1 et
- les composants (b1) et (b2) dans un rapport de poids de 80:20 à 20:80
sont présents.

2. Préparation selon la revendication 1, **caractérisée par le fait que** les composés de la formule (I) sont choisis dans le groupe formé par l'eugénol, l'anéthole, le thymol et le carvacol et leurs mélanges.

3. Préparations pour administration orale contenant les mélanges selon la revendication 1.

4. Préparations selon la revendication 3, **caractérisées en ce qu'**elles sont des denrées alimentaires, des préparations pharmaceutiques et/ou des préparations de soins bucco-dentaires.

5. Préparations selon la revendication 3, **caractérisées par le fait qu'**elles sont des bonbons durs, des bonbons à mâcher ou des chewing-gums.

6. Préparations selon la revendication 3, **caractérisées par le fait qu'**il s'agit de jus froids, de sprays froids ou de pastilles froides.

7. Préparations selon la revendication 3, **caractérisées par le fait qu'**il s'agit de dentifrices, de bains de bouche ou de gommes à mâcher médicinales.

8. Préparations selon l'une au moins des revendications 3 à 7, **caractérisées en ce qu'**elles contiennent les préparations selon la revendication 1 en quantités de 0,5 à 5% en poids - par rapport à la préparation finale.

9. Procédé pour renforcer le goût épicé et chaud et réduire le goût brûlant et douloureux des huiles essentielles contenant des composés de la formule (I) (composant a), **caractérisé en ce qu'**un mélange de menthol (composant b1) et d'acétate de menthyle (composant b2) est ajouté aux préparations contenant ces substances, dans lequel dans ladite huile essentielle
- les composants (a) et (b1+b2) dans un rapport en poids de 80:20 à 99:1 et
- les composants (b1) et (b2) dans un rapport de poids de 80:20 à 20:80
sont présents.

10. Utilisation d'un mélange de menthol (composant b1) et d'acétate de menthyle (composant b2) pour renforcer le goût épicé et chaud et réduire le goût brûlant et douloureux des huiles essentielles contenant des composés de la formule (I) (composant a), dans laquelle dans ladite huile essentielle
- les composants (a) et (b1+b2) dans un rapport de poids de 80:20 à 99:1 et
- les composants (b1) et (b2) dans un rapport de poids de 80:20 à 20:80
sont utilisés.
